# EUROPEAN PATENT SPECIFICATION

(11) **EP 1 568 705 B1**
(45) Date of publication and mention of the grant of the patent: **29.10.2008**
(21) Application number: 03811939.2
(22) Date of filing: 27.11.2003
(51) Int. Cl.: C12N 15/10, C07H 21/00, G01N 27/447

(54) **METHOD AND APPARATUS FOR CONCENTRATION AND PURIFICATION OF NUCLEIC ACID**
VERFAHREN UND APPARAT ZUM ANREICHERN UND ZUR AUFREINIGUNG VON NUKLEINSÄURE
PROCEDE ET APPAREIL DE CONCENTRATION ET DE PURIFICATION D'ACIDE NUCLEIQUE

(30) Priority: 28.11.2002 JP 2002345210; 10.11.2003 JP 2003379796
(43) Date of publication of application: 31.08.2005
(73) Proprietor: ARKRAY INC., Kyoto-shi, Kyoto 601-8045 (JP)
(72) Inventor: HIRAI, Mitsuharu, c/o ARKRAY INC., Kyoto-shi, Kyoto 601-8045 (JP); MURAKAMI, Jun, c/o ARKRAY INC., Kyoto-shi, Kyoto 601-8045 (JP); HASHIGUCHI, Satoshi, c/o ARKRAY INC., Kyoto-shi, Kyoto 601-8045 (JP); INOSE, Ken, c/o ARKRAY INC., Kyoto-shi, Kyoto 601-8045 (JP)
(74) Representative: Jostarndt, Hans-Dieter
(86) International application number: PCT/JP2003/015132
(87) International publication number: WO 2004/048398

(56) References cited:
- EP-A- 0 853 123
- EP-A- 1 696 026
- EP-A2- 0 979 868
- WO-A-00/71999
- WO-A-98/10277
- WO-A-99/38874
- WO-A-20/04027080
- WO-A1-00/71999
- WO-A1-98/10277
- WO-A2-99/38874
- DE-A1- 3 626 953
- GB-A- 2 148 325
- JP-A- 8 327 595
- US-A- 5 275 708
- JACQUIER J C ET AL: "Capillary electrophoretic study of the complex formation between DNA and cationic surfactants." JOURNAL OF CHROMATOGRAPHY. A. 21 AUG 1998, vol. 817, no. 1-2, 21 August 1998 (1998-08-21), pages 263-271, XP002410343 ISSN: 0021-9673
- BHATTACHARYA S ET AL: "Interaction of surfactants with DNA. Role of hydrophobicity and surface charge on intercalation and DNA melting." BIOCHIMICA ET BIOPHYSICA ACTA. 14 JAN 1997, vol. 1323, no. 1, 14 January 1997 (1997-01-14), pages 29-44, XP002410344 ISSN: 0006-3002
- DATABASE WPI Week 199540 Derwent Publications Ltd., London, GB; AN 1995-307165 XP002410366 & JP 07 203965 A (CANON KK) 8 August 1995 (1995-08-08)
- DATABASE WPI Week 199709 Derwent Publications Ltd., London, GB; AN 1997-090499 XP002422666 & JP 08 327595 A (TOYO ROSHI KK) 13 December 1996 (1996-12-13)

## Description

### Field of the Invention

The present invention relates to a method for separation of a nucleic acid. In more detail, the present invention relates to a method for separation of a target nucleic acid by electrophoresis.

### Background Art

Due to the recent decipherment of the human genome, various relations between life processes and genes have been analyzed. Consequently, medical importance shifts from pathology to etiology, and from medical treatment to prevention. Hereupon, the gene test technology becomes an important basis.

The gene test can be used for tests which are difficult to be carried out by the conventional clinical examination, such as identification of pathogenic microorganisms difficult to be cultivated, detection of pathogenic microorganisms under medical treatment with antibiotics or at an early stage of infection, detection of antigens in case of suspect of existence of transferred antibody, investigation of a source of infection of pathogenic microorganisms, personal identification such as parentage diagnosis, gene diagnosis of disease type of leukemia and solid tumor, and established diagnosis of genetic disease. Bacteria, which requires a long time for cultivation thereof, can be effectively detected by the gene test, because the gene test takes a short time compared with a method using cultivation of bacteria. Furthermore, since DNA is stable depending on a good storage condition, an old sample, such as a frozen biopsy or a bone, also can be tested.

The gene test attracts public attention because it can expand opportunities of test of recently increasing sexual transmitted diseases.

There are well-known conventional methods of purification and concentration of a nucleic acid, including a purification method using phenol, chloroform or ethanol, a purification method using a column or filter which adsorbs nucleic acids, and a purification method using magnetic silica beads.

Furthermore, there is a well-known conventional method for recovery of a nucleic acid from a plate-like electrophoresis gel, as described in the Japanese Utility Model Laid Open Gazette Hei. 5-88296, whereby nucleic acids are electrophoresed in a prepared gel, a recovery device is moved to a portion of the gel involving target nucleic acids, and then the target nucleic acids are further electrophored to be recovered.

Furthermore, there is a well-known conventional method as described in the Japanese Patent Laid Open Gazette Hei. 8-327595, whereby nucleic acids are electrophoresed in a plate-like electrophoresis gel so as to separate target nucleic acids, and a recovery chip is inserted into the gel near a band of the target nucleic acids so as to recover the target nucleic acids.

With regard to the conventional methods for purification and concentration of a nucleic acid, the purification method using phenol, chloroform or ethanol is available in only limited environments because it needs a powerful medicine requiring a highly advanced chemical equipment. Further, the purification method is difficult to be automated because it requires laborious operations and high-speed centrifugation process. It is also difficult to obtain high refining accuracy.

The purification method using a column or filter for adsorbing nucleic acids is performed while flowing solution. Therefore, when a sample includes many impurities such as rubbish, the column or filter tends to clog so as to reduce purification efficiency. Furthermore, the method is difficult to be automated because it requires centrifugation or aspiration process.

The recovery method using magnetic silica beads is difficult to obtain high recovery of nucleic acid, because a silica bead, which is failed in recovery by a magnet or falls off from magnetic material, may remain in a sample.

The conventional method for recovering a nucleic acid from a plate-like electrophoresis gel requires the plate-like electrophoresis gel and the electrophoresis of nucleic acids in the plate-like electrophoresis gel before processing the portion of the gel involving the target nucleic acids.

The gel for electrophoresis is weak against a shock, and may change in characteristic according to formation process thereof. Therefore, generally, the position of the target nucleic acids in the electrophoresis gel is analyzed by ultraviolet rays after electrophoresis, and subsequently, the portion of the gel containing high content of the target nucleic acids is processed.

Consequently, the gene test using this method takes a long time per unit of test. If the gel for electrophoresis is large, blot of the band of the nucleic acids caused by unevenness of the gel is increased, thereby reducing recovery of the nucleic acids. Furthermore, the large gel requires large electric power for the electrophoresis.

### Summary of the Invention

As a result of various examinations to solve the above-mentioned problems, a method is found that surfactant is added into a sample so as to adsorb an impurity in the sample, thereby making migration of the impurity different from migration of an nucleic acid, and separating the impurity from the nucleic acid.

Furthermore, the impurity, excluding the nucleic acid, is electrically charged by cationic surfactant and nonionic surfactant and placed in an electric field, whereby the nucleic acid is separate and purified from the sample containing the impurity so as to be concentrated or easy to be concentrated.

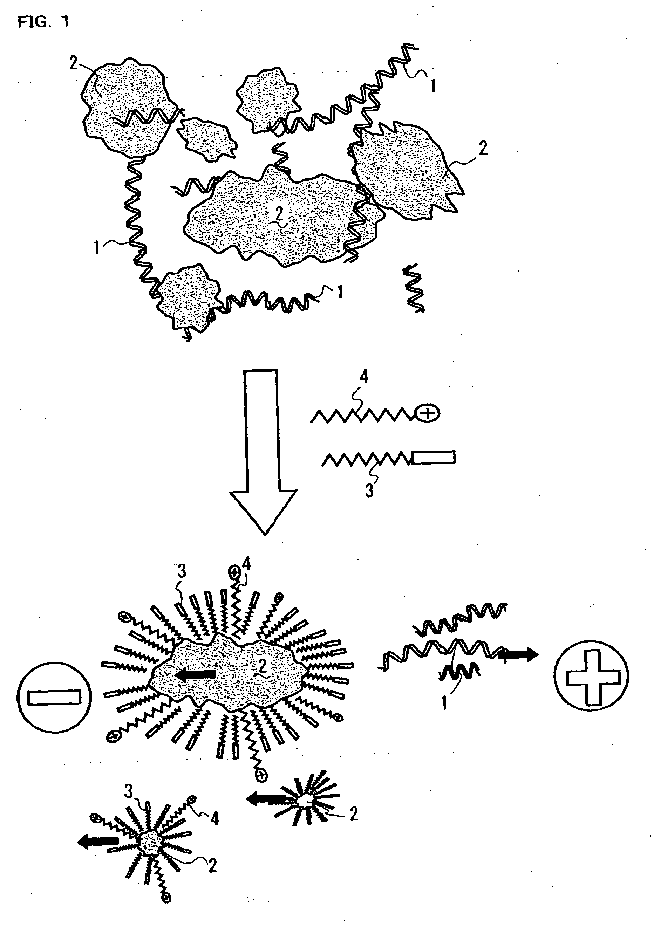

Fig. 1 is a mimetic diagram of concentration of nucleic acids by electrophoresis under the existence of surfactant. A sample contains nucleic acids 1 and impurities 2. Surfactant is added to the sample. Nonionic surfactant 3 and cationic surfactant 4 are used as the surfactant. The surfactants mixed with the sample adsorb the impurities 2. The impurities 2 adsorbed by the cationic surfactant 4 are charged in positive, thereby being separated from the nucleic acids 1 which are little or not adsorbed by the surfactant. Accordingly, the impurities 2 can be easily removed from the sample so as to concentrate the nucleic acids 1.

Next, explanation will be given of a method for concentration of nucleic acids. In this method, electrophoresis is performed twice so as to surely concentrate nucleic acids. Excessive ions in the sample are removed by the first electrophoresis, and the nucleic acids in the sample are concentrated by the second electrophoresis. Firstly, 100µL of 1% Triton (registered trademark) X-100, as the nonionic surfactant, is added to the sample containing the nucleic acids, and mixed, and then heated at 96°C for 10 minutes. Then, 100µL of 0.2% DPC, as the cationic surfactant, is added to the sample. Alternatively, both the nonionic surfactant and the cationic surfactant may be simultaneously added before the heat treatment. Even if the nucleic acids exist in procaryotic cells of colon bacillus or the like, cell walls are destroyed by the surfactants which are added to the sample for pretreatment. Accordingly, culture solution of colon bacillus or the like can be used as the sample, thereby facilitating the operation of pretreatment of the sample. The pretreatment is performed as the above, and direct voltage of 100V is applied so as to perform electrophoresis for 10 minutes, thereby removing the excessive ions from the sample. Subsequently, direct voltage of 125 to 150V is applied so as to perform electrophoresis for 120 minutes, thereby recovering the nucleic acids at the side of positive electrode.

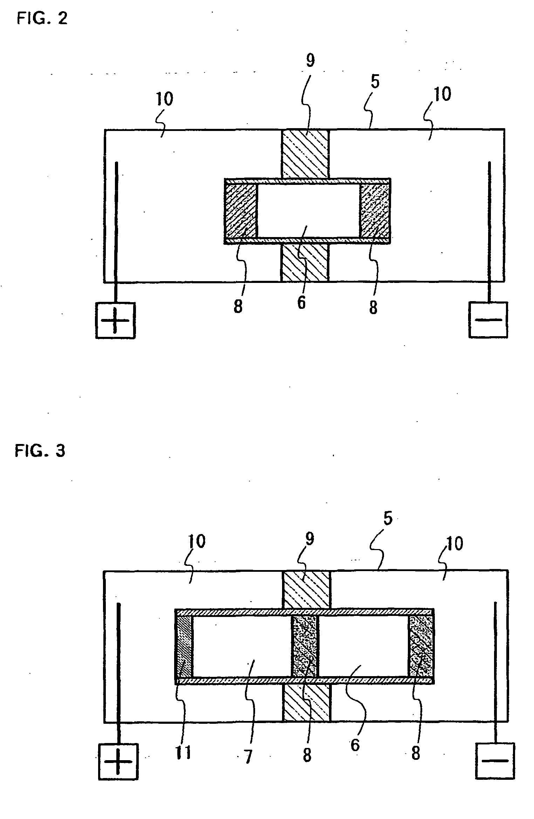

Explanation will be given of a construction of an electrophoresis tank for the electrophoresis of the sample. With regard to the first electrophoresis, Fig. 2 shows a construction of an electrophoresis tank 5 for the first electrophoresis. The electrophoresis tank 5 is provided therein with a sample tank 6 and a partition 9 dividing the electrophoresis tank 5 into a positive electrode side part and a negative electrode side part. The sample tank 6 penetrates the partition 9, and is projected at one side thereof toward the positive electrode, and at the other side thereof toward the negative electrode. The sample tank 6 has opposite open ends closed with respective gels 8. Potential difference is generated in the sample tank 6 so as to electrophorese the nucleic acids and the impurities adsorbed by the surfactant. Both the nonionic surfactant and the cationic surfactant are added to the sample, and the sample is heated. Then, the sample is injected into the sample tank 6. Voltage of 100V is applied across the electrodes so as to perform electrophoresis for 10 minutes. Accordingly, the excessive ions are removed from the sample. After removing the excessive ions, the nucleic acids are concentrated by the second electrophoresis.

With regard to the second electrophoresis, the sample tank containing the sample injected at the first electrophoresis process is connected to a recovery tank and disposed in an electrophoresis tank so as to perform the second electrophoresis. Fig. 3 shows a construction of the electrophoresis tank 5 for the second electrophoresis. The electrophoresis tank 5 is provided therein with the sample tank 6, the recovery tank 7 and a partition 9 dividing the electrophoresis tank 5 into a positive electrode side part and a negative electrode side part. The sample tank 6 is inserted into the partition 9 and projected to the negative electrode side. The recovery tank 7 is inserted into the partition 9 and projected to the positive electrode side. The sample tank 6 and the recovery tank 7 are connected with each other in the partition 9 through a gel 8. The sample tank 6 has opposite open ends closed with respective gels 8. The recovery tank 7 has opposite open ends. One of the ends of the recovery tank 7 on the negative electrode side is closed with the gel 8, and the other end of the recovery tank 7 on the positive electrode side is closed with an ultrafiltration film 11. In the electrophoresis tank constructed as the above, voltage of 120V is applied across the electrodes so as to perform electrophoresis for 120 minutes.

By the second electrophoresis in the electrophoresis tank, the sample after removal of the excessive ions is electrophoresed at a part thereof excluding the nucleic acids, whereby the nucleic acids can be efficiently recovered into the recovery tank 7. The ultrafiltration film 11 provided on the positive electrode side of the recovery tank 7 prevents the nucleic acids from leaking out from the recovery tank 7. Accordingly, recovery efficiency of the nucleic acids can be improved. If a state of a sample is suitable, the only second electrophoresis can be performed without performing the first electrophoresis. A pretreated sample is injected into the sample tank 6 connected to the recovery tank 7 so as to be electrophoresed, whereby the nucleic acids can be concentrated easily. The excessive ions remaining in the sample can pass through the ultrafiltration film, thereby being prevented from influencing the recovery of the nucleic acids.

Namely, according to the present invention, a method for concentration and purification of a nucleic acid using electrophoresis is characterized in that a sample is placed in an electric field so as to concentrate and purify the nucleic acids after adjusting charge amount of an impurity in the sample containing the nucleic acid. Further, according to the present invention, a method for concentration and purification of a nucleic acid using electrophoresis is characterized in that cationic surfactant is added to a sample containing a nucleic acid so as to adjust charge amount of an impurity in the sample, and then the sample is placed in an electric field so as to concentrate and purify the nucleic acid by the electrophoresis. Cationic surfactant and nonionic surfactant are added to a sample containing a nucleic acid so as to adjust charge amount of an impurity in the sample, and then the sample is placed in an electric field so as to concentrate and purify the nucleic acids by the electrophoresis. The electric charge of substance other than the nucleic acid in the sample is adjusted by adsorption thereof to the cationic surfactant, and the adsorption of the substance to the cationic surfactant is adjusted by adjusting an amount of the added nonionic surfactant.

According to the present disclosure an apparatus for concentration and purification of a nucleic acid comprises cationic surfactant and nonionic surfactant added to a sample, wherein the sample is electrophoresed so as to concentrate and purify the nucleic acid at a positive electrode side. Further, the apparatus comprises a container whose side wall is formed of an insulator. A conductive partition member for preventing diffusion is provided in the container so as to divide the interior of the container into a sample introduction chamber and a nucleic acid recovery chamber. Each of ends of the container is connected to an electrode through a buffer tank, respectively.

Alternatively, according to the present invention following means may be used. A sample containing nucleic acids is contacted with a separation medium formed of material in which substances having different molecular weights become different in mobility, and voltage is applied so as to separate the nucleic acids from the other substances. Then, the nucleic acids passed through the separation medium are recovered by a filter. The filter has pores for passing nucleic acids which are smaller than the target nucleic acids.

For example, the separation medium used for electrophoresis is provided on one end thereof with a sample supply part filled with buffer, and on the other end thereof with a sampling part filled with buffer. By applying voltage on the sample supply part and the sampling part, the sample is electrophoresed to migrate in the separation medium. Due to different molecular weights of the respective nucleic acids, the nucleic acids in the sample migrate in the separation medium at different speeds, thereby resulting in that the nucleic acids having different molecular weights require different times to pass though the separation medium and be eluted into the sampling part. In consideration of this situation, the electrophoresis is stopped immediately after the end of elution of the target nucleic acids into the sampling part, so as to reduce components excluding the target nucleic acids. The buffer in the sampling part can be exchangeable corresponding to the time for the target nucleic acids to elute into the sampling part, so as to sample only the target nucleic acids from the sampling part. Concentration of the target nucleic acids depends on the amount of the buffer in the sampling part.

In this way, the above operation can be easily automated because it does not require centrifugation or aspiration process. Nucleic acids can be separated from a sample with large volume at once. By reducing the eluted solution containing the target nucleic acids, the solution is concentrated so as to increase concentration of the target nucleic acids in the sample. Further, such nucleic acids purified to be easily treated are sampled immediately after the electrophoresis, thereby smoothening connection of the present process to a next process.

According to the present method, compared to other methods, higher yield and higher accuracy of the target can be obtained, and expensive reagent or an expensive apparatus is not required, thereby reducing running cost and cost for the operation. In this regard, according to the present method for concentrate and purify of a nucleic acid using electrophoresis, an impurity in a sample containing nucleic acids is contacted with a separation medium, in which the electrophoresed nucleic acids having different molecular weights migrate at different rates corresponding to the difference of molecular weights, and then, a nucleic acid smaller than a target nucleic acid pass through a filter having a sectional area reduced in the direction of migration and the target nucleic acid is recovered by the filter, thereby separating the target nucleic acid from the sample. The method can be performed without centrifugation or aspiration operation, and can use a simple apparatus for separation and concentration of a nucleic acid. Further, such a simple construction can be easily automated. Further, a large quantity of sample can be obtained at once. The eluted solution containing the target nucleic acid can be reduced for concentration so as to increase concentration of the target nucleic acid in the sample. Running cost for the separation and concentration and time for the operation can be reduced. Moreover, a test for identifying an exogenous gene which does not originally exist can be performed easily.

### Brief Description of the Drawings

Fig. 1 is a mimetic diagram of concentration of nucleic acids by electrophoresis under the existence of surfactant.
Fig. 2 is a diagram of an electrophoresis tank for first electrophoresis.
Fig. 3 is a diagram of an electrophoresis tank for second electrophoresis.
Fig. 4 is a diagram of a first electrophoresis tank.
Fig. 5 is a diagram of a second electrophoresis tank.
Fig. 6 is a perspective view of a sampling unit.
Fig. 7 is a plane view of the sampling unit.
Fig. 8 is a side view of the sampling unit.
Fig. 9 is a sectional side view of the sampling unit.
Fig. 10 is a perspective view of a connection part.
Fig. 11 is a sectional side view of the connection part.
Fig. 12 is a sectional side view of a filter part.
Fig. 13 is a side view of assembly of a separation unit.
Fig. 14 illustrates a UV spectrum of recovered solution.
Fig. 15 is a perspective view partially in section of a nucleic acid concentration unit.
Fig. 16 is a plane view of the nucleic acid concentration unit.
Fig. 17 is a side view of the nucleic acid concentration unit.
Fig. 18 illustrates concentration processes of target nucleic acids.
Fig. 19 is a diagram of opening processes of the concentration unit.
Fig. 20 is a sectional side view of the concentration unit.

### Best Mode for Carrying out the Invention

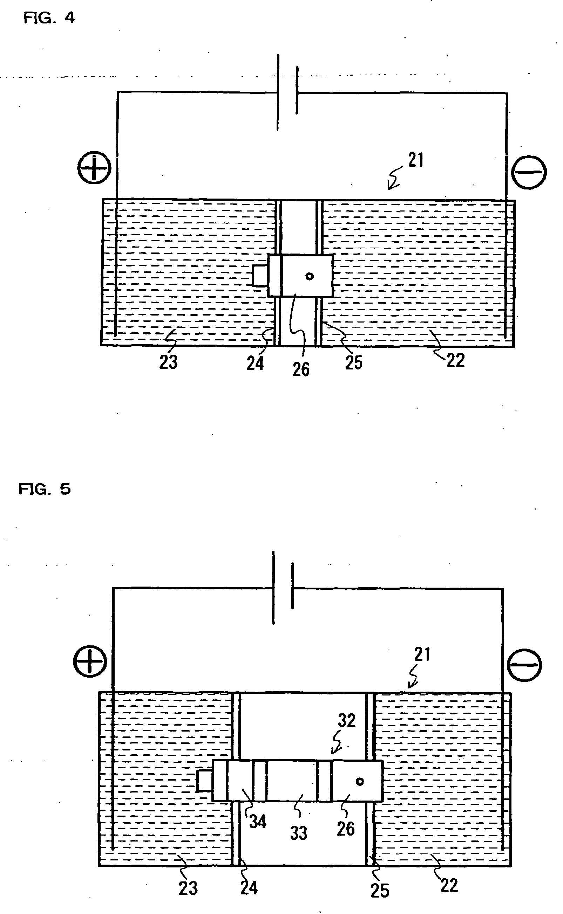

Firstly, an electrophoresis tank for electrophoresis will be described. Fig. 4 illustrates a first electrophoresis tank 21. An electrophoresis tank 21 is divided into a negative electrode side tank 22 and a positive electrode side tank 23 by partitions 24 and 25. The partitions 24 and 25 are disposed at a center of the electrophoresis tank 21, and a sampling unit 26 is attached to the partitions 24 and 25. One end of the sampling unit 26 is projected into the negative electrode side tank 22, and the other end thereof is projected into the positive electrode side tank 23. A positive electrode side portion of the sampling unit 26 is filled with gel. A negative electrode side portion of the sampling unit 26 is filled with gel, and is provided in its side surface with an injection hole through which a sample is injected into the sample unit. At the time of electrophoresis, the injection hole is shut with a plug. A negative electrode is inserted into the negative electrode side tank 22 and a positive electrode is inserted into the positive electrode side tank 23 so as to apply voltage to the electrophoresis tank 21.

Another electrophoresis tank will be described. Fig. 5 illustrates a second electrophoresis tank 21. The second electrophoresis tank 21 is divided into a negative electrode side tank 22 and a positive electrode side tank 23 by partitions 24 and 25. The partitions 24 and 25 are disposed at a center of the electrophoresis tank 21, and a separation unit 32 is attached to the partitions 24 and 25. One end of the separation unit 32 is projected into the negative electrode side tank 22, and the other end thereof is projected into the positive electrode side tank 23. A negative electrode is inserted into the negative electrode side tank 22 and a positive electrode is inserted into the positive electrode side tank 23 so as to apply voltage to the electrophoresis tank 21. The separation unit 32 comprises three units, i.e., a sampling unit 26, a connection part 33 and a filter part 34, which are connected to one another. O-rings are interposed between the sampling unit 26 and the connection part 33, and between the connection part 33 and the filter part 34 so as to secure their connection and prevent outflow of buffer solution. A negative electrode side portion of the sampling unit 26 is filled with gel, and a positive side portion of the connection part 33 also is filled with gel. An ultrafiltration membrane is attached to the filter part 34.

Nucleic acids are concentrated in such a electrophoresis tank. Firstly, with regard to the first electrophoresis, a dissolved sample is injected through the injection hole and the hole is plugged. Then, the sampling unit 26 is installed in the electrophoresis tank 21 so that an upper surface of the sampling unit 26 slightly projects upward from the solution. Subsequently, direct voltage of 100V is applied and electrophoresis is performed for 20 minutes, thereby removing excessive ions from the sample. The buffer solution is prepared from 1x TAE solution, 40mM Tris, 40mM glacial acetic acid, and 1mM EDTA, and pH thereof is adjusted to be 8.0.

After removing excessive ions from the first electrophoresis tank 21, the connection part 33 and the filter part 34 are connected to the sampling unit 26. An O-ling is interposed in each junction so as to prevent leak of the solution. A solution prepared by mixing 100% ethanol and 1x TAE with the mixture ratio of 6:4 is supplied into the connection part 33, and TE-1 (10mM Tris-HCl, 0.1mM EDTA, pH 8.0) is supplied into the filter part 34.

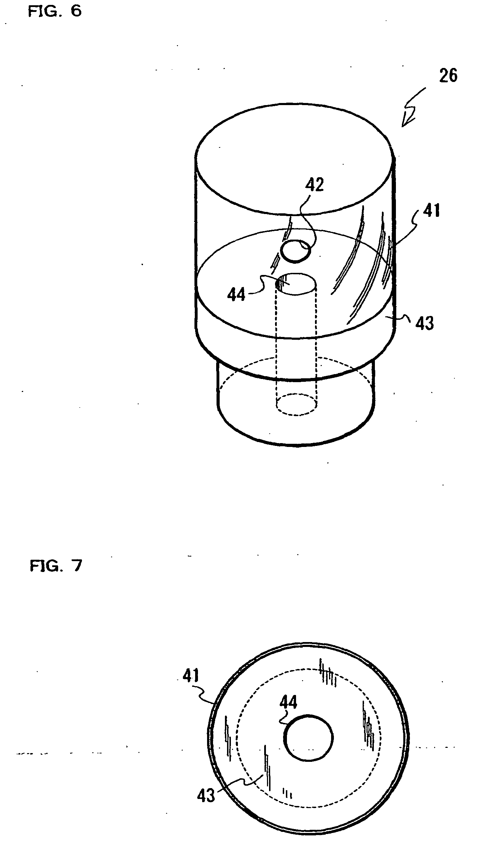

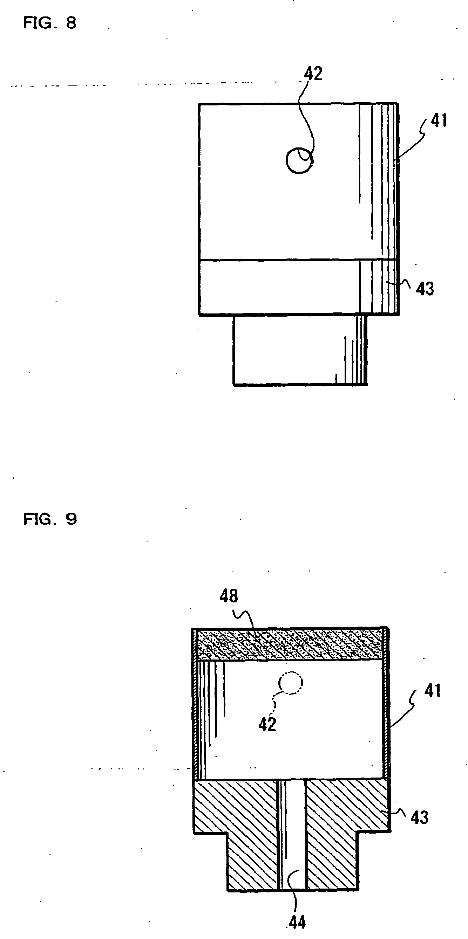

Next, explanation will be given of constructions of the sampling unit 26, the connection part 33 and the filter part 34. Firstly, the construction of the sampling unit 26 will be described. Fig. 6 is a perspective view of the sampling unit, Fig. 7 is a plane view of the sampling unit, Fig. 8 is a side view of the sampling unit, and Fig. 9 is a sectional side view of the sampling unit. The sampling unit 26 comprises a container for containing gel. The container is Microcon (registered trademark) YM-3 centrifugal filter unit (Millipore) processed so that an ultrafiltration membrane is removed therefrom and that a hole having a 5mm diameter is opened therein. Alternatively, any unit can be used as the sampling unit 26, if it has the same effect.

The sampling unit 26 comprises a cylinder body 41 and a base 43. The cylinder body 41 is connected to the base 43 and provided therein with an injection hole 42 for injecting a sample. The base 43 is formed to be a stepped column, and a vertical hole 44 penetrates the base 43. Gel 48 is disposed in the cylinder body 41. The gel 48 has thickness of several mm such as to fill an opening of the cylinder body 41. Accordingly, a sample is supplied through the injection hole 42 inside the gel 48.

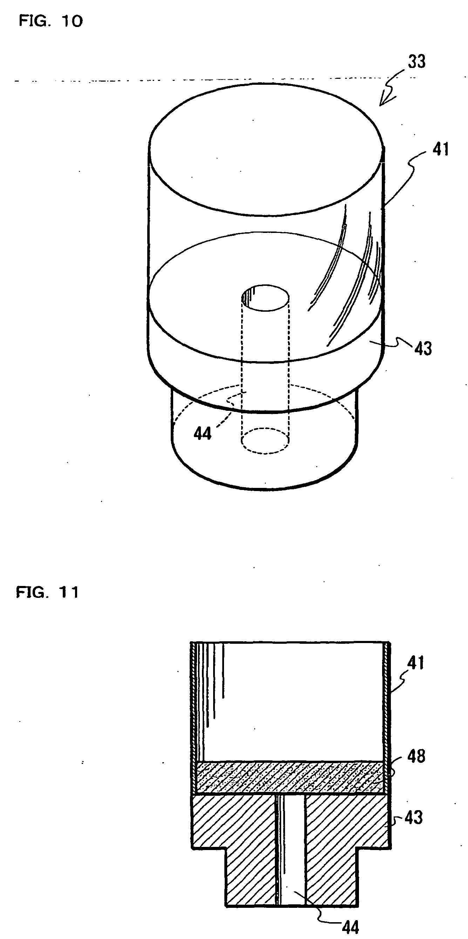

Explanation will be given of the connection part 33. Fig. 10 is a perspective view of the connection part, and Fig. 11 is a sectional side view of the connection part. Similar to the sampling unit 26, a centrifugal filter unit of Millipore is processed so that that an ultrafiltration membrane is removed therefrom and a hole having a 5mm diameter is opened therein, thereby constructing the connection part 33. The connection part 33 comprises a cylinder body 41 and a base 43. The cylinder body 41 is connected to the base 43. The base 43 is formed to be a stepped column, and a vertical hole 44 penetrates the base 43. Gel 48 having thickness of several mm is disposed in the cylinder body 41. In the cylinder body 41, the gel 48 is disposed on an upper surface of the base 43 so as to prevent liquid from flowing from/to the filter part 34.

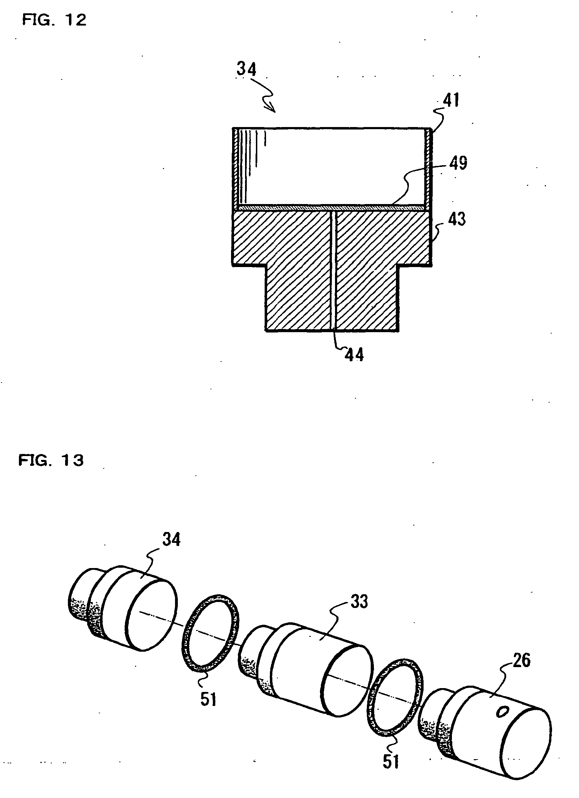

Explanation will be given of the filter part 34. Fig. 12 is a sectional side view of a filter part. The filter part 34 also is constructed by processing a centrifugal filter unit of Millipore. The filter part 34 comprises a cylinder body 41 and a base 43. The cylinder body 41 is connected to the base 43. This cylinder body 41 is about 5mm shorter than the cylinder bodies 41 of the sampling unit 26 and the connection part 33. The base 43 is formed to be a stepped column, and a vertical hole 44 penetrates the base 43. In the cylinder body 41, an ultrafiltration membrane 49 is disposed in an upper surface of the base 43 so as to prevent nucleic acids from flowing out, thereby ensuring concentration of nucleic acids.

Next, explanation will be given of an embodiment of the concentration operation of nucleic acids. A sample is culture solution of colon bacillus, and the first and second electrophoreses are performed so as to concentrate the nucleic acids. The concentration of the recovered nucleic acids is measured by absorbance measurement. 100µL of culture solution of colon bacillus (*Escherichia coli* DH5 α) is used as the sample. 100µL of 1% Triton (registered trademark) X-100 is added to the sample, and is heated at 96°C for 10 minutes. Subsequently, 100µL of 0.2% DPC is added thereto, thereby preparing the sample for the electrophoresis. 0.5x TAE is used as buffer for the electrophoresis. Agarose is dissolved in 1x TAE. In addition, the 1x TAE solution is prepared from 40mM Tris, 40mM glacial acetic acid, and 1mM EDTA, and pH thereof is adjusted to be 8.0.

The connection part 33 is stood so as to turn the opening of the cylinder body 41 upward, and 1% agarose gel (SeaKem Gold agarose: bought from TaKaRa) is supplied into the cylinder body 41 through the opening so as to make the gel have several mm thickness, and then, the gel is hardened. Similar to the connection part 33, the sampling unit 26 is stood so as to turn the opening of the cylinder body 41 upward, and 1% agarose gel (SeaKem Gold agarose: bought from TaKaRa) is supplied into the cylinder body 41 through its opening so as to make the gel have several mm thickness. After the gel is hardened, the sampling unit 26 is reversed and the gel is supplied into the opening of the cylinder body 41 with an injection hole 42. HU-6 (made by AR BROWN) is used as the electrophoresis tank, and MPSU-200 (made by AR BROWN) is used as a power source.

The prepared sample for the electrophoresis is supplied into the sampling unit 26 through the injection hole 42, and then the hole is plugged. The electrophoresis tank is divided into the negative electrode side part and the positive electrode side part by putty, and 0.5x TAE is supplied into both side parts in the tank. The sampling unit 26 is disposed to the putty so as to make the upper surface of the sampling unit 26 upper than the buffer solution. Subsequently, direct voltage of 100V is applied and the first electrophoresis is performed for 20 minutes.

The connection part 33 and the filter part 34 are connected to the sampling unit 26 after finishing the first electrophoresis so as to construct the separation unit, and the second electrophoresis is performed.

Explanation will be given of an embodiment of operation for the second electrophoresis. A solution is prepared by mixing 100% ethanol and 1x TAE with the mixture ratio of 6:4, and is supplied into the connection part 33. TE-1 (10mM Tris-HCl, 0.1mM EDTA, pH 8.0) is supplied into the filter part 34.

Next, the connection part 33 and the filter part 34 are connected to the sampling unit 26 so as to assemble the separation unit. Fig. 13 is a side view of the assembly construction of the separation unit. The separation unit is assembled from the sampling unit 26, the connection part 33 and the filter part 34, which are turned in the same direction. O-rings 51 are interposed between the connection part 33 and the filter part 34, and between the connection part 33 and the filter part 34 so as to prevent leak of the solution from the separation unit.

The electrophoresis tank is divided into the negative electrode side part and the positive electrode side part by putty, and 0.5x TAE is supplied into both sides of the tank. The assembled separation unit is disposed so that the sampling unit 26 is disposed on the negative electrode side of the putty and the filter part 34 on the positive electrode side of the putty. Subsequently, direct voltage of 200V is applied and the second electrophoresis is performed for 240 minutes.

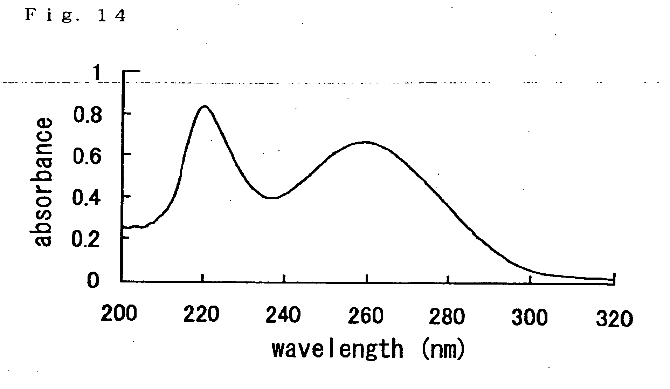

Next, the nucleic acid solution is recovered at the filter part 34 and its absorbance is measured by UV spectrum so as to calculate the concentration of the recovered nucleic acids. Fig. 14 illustrates a UV spectrum of the recovered solution. The calculated concentration of the nucleic acids is 32.3ng/µL (6.7*10⁶ copies/µL). The concentration of the nucleic acids is calculated so that the absorbance at 260nm (A260) is multiplied by a coefficient peculiar to the property of the nucleic acids, multiplied by an optical path length of a cell (mm), and divided by 10.

The purity of the recovered nucleic acids is calculated by the UV spectrum. The calculated purity of the recovered nucleic acids is 1.91. The purity is calculated by dividing the absorbance at 260nm (A260) by the absorbance at 280nm (A280). When the sample is DNA with the purity of 100%, calculated value is about 1.8. When the sample is RNA with the purity of 100%, calculated value is about 2.0. Amount of proteins and phenols mixed in measured material is reflected in the value of A280. When the absorbance ratio is much less than 1.5, it is conceivable that monomeric substances, such as proteins, are mixed.

According to a method for concentration and purification of a nucleic acid using electrophoresis, electric charge of an impurity in a sample containing a nucleic acid is adjusted, and then the sample is placed in an electric field for electrophoresis so as to concentrate and purify the nucleic acid. As an effect of the method, migration of the nucleic acid is different from migration of the impurity at the time of the electrophoresis so as to separate the nucleic acid efficiently. The difference of migration between the impurity and the nucleic acid is adjustable by the adjustment of the electric charge, whereby the migration can be controlled easily. It is not necessary to use a centrifugal separator or the like, whereby the mechanism for concentrating the nucleic acid can be compacted.

According to a method for concentration and purification of a nucleic acid using electrophoresis, cationic surfactant is added to a sample containing a nucleic acid so as to adjust the electric charge of the impurity in the sample, and then the sample is placed in an electric field for electrophoresis so as to concentrate and purify the nucleic acid. Due to the cationic surfactant, an operation for the method is easy, and the safety of the operation is easily secured. By increasing the adsorption to the impurity, the difference of migration between the impurity and the nucleic acid becomes large so as to separate the nucleic acids efficiently. The impurity migrates in the direction opposite to the nucleic acid so as to prevent the impurity from inhibiting purification of the nucleic acid, thereby easily purifying the nucleic acid.

According to a method for concentration and purification of a nucleic acid using electrophoresis, cationic surfactant and nonionic surfactant are added to a sample containing a nucleic acid so as to adjust electric charge of an impurity in the sample, and then the sample is placed in an electric field for electrophoresis so as to concentrate and purify the nucleic acid. Accordingly, the adsorption of the cationic surfactant can be adjusted by adjusting a ratio between the cationic surfactant and the nonionic surfactant, thereby easily adjusting migration of the electrophoresed impurity

The electric charge of substance other than the nucleic acid is adjusted by the adsorption thereof the cationic surfactant, and the adsorption is adjusted by the added amount of the nonionic surfactant. Accordingly, the adsorption ratio of the impurity to the cationic surfactant and the ratio of the nonionic surfactant can be operated easily. Furthermore, the electric charge of the impurity can be adjusted by easy operation.

In an apparatus, cationic surfactant and nonionic surfactant are added to a sample, and the sample is electrophoresed so as to concentrate and purify the nucleic acid at a positive electrode side. Accordingly, the apparatus can be simplified to purify the nucleic acid in the sample and to control migration of the nucleic acid, thereby being compacted while securing safety thereof.

An apparatus for concentration and purification of a nucleic acid using electrophoresis comprises a container, whose side wall is formed of an insulator. The container is divided into a sample introduction chamber and a nucleic acid recovery chamber by a conductive separation medium for preventing diffusion. Ends of the container are connected to respective electrodes through respective buffer tanks. Accordingly, the apparatus for concentration and purification can be simply constructed so as to reduce its production cost. Furthermore, the apparatus can be controlled easily and has high safety.

Next, explanation will be given to the drawings.

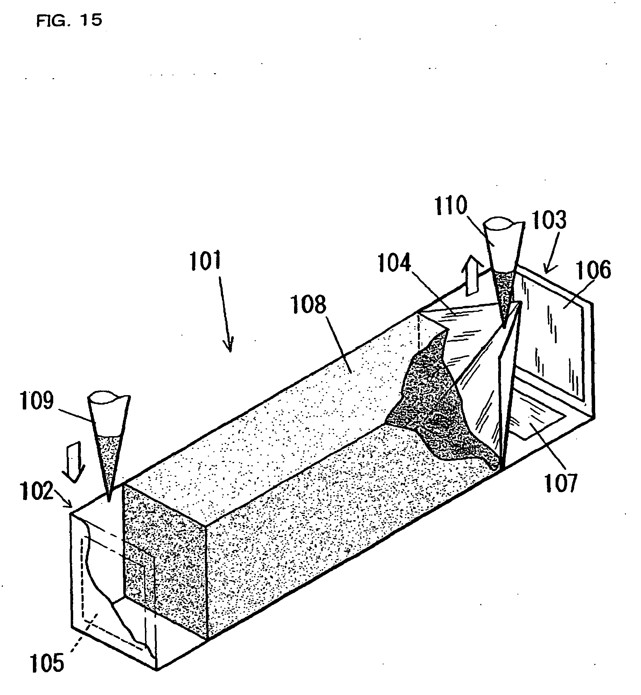

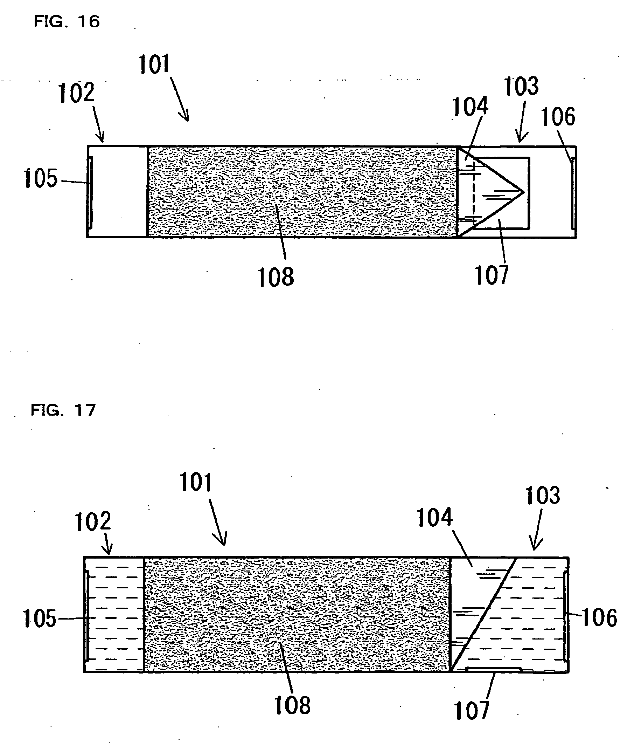

Fig. 15 is a perspective view partially in section of a nucleic acid concentration unit, Fig. 16 is a plane view of the unit, and Fig. 17 is a side view of the unit. The nucleic acid concentration unit shown in Figs. 15 to 17 will be described.

The concentration unit 101 in a t-DNA detector separates and concentrates target nucleic acids. The concentration unit 101 comprises an injection chamber 102, a separation chamber 108 formed of a separation medium, and a sampling chamber 103. A sample containing the target nucleic acids is introduced into the injection chamber 102 through a nozzle 109, and voltage is impressed on the injection chamber 102 and the sampling chamber 103 so as to make the nucleic acids migrate into the sampling chamber 103. The target nucleic acids having passed through the separation chamber 108 elute into the sampling chamber 103, and are sampled from the sampling chamber 103 through a nozzle 110. A biological sample, such as blood, urine, sputum or the like, a beverage, or a food can be used as the sample to be injected into the injection chamber 102. In addition, genome or plasmid can be injected.

Construction of each part in the concentration unit 101 will be described in detail. The injection chamber 102 is connected to one end of the separation chamber 108, and the sampling chamber 103 is connected to the other end of the separation chamber 108. An electrode 105 is disposed in the injection chamber, and electrodes 106 and 107 are disposed in the sampling chamber 103. The injection chamber 102 and the sampling chamber 103 are filled with buffer for the electrophoresis. The electrode 106 in the sampling chamber 103 is positioned so as to face to the electrode in the injection chamber 102, and the electrode 107 in the sampling chamber 103 is positioned on a bottom of the sampling chamber 103. A filter 104 is disposed in the sampling chamber 103 so as to divide the sampling chamber 103 into the separation chamber 108 and the chamber including the electrode 106. The filter 104 permits substance smaller than the target nucleic acids to pass therethrough, and prevents the substance not smaller than the target nucleic acids from passing therethrough. The filter 104 is provided therein with many pores having characteristic such as to be prevented from interacting with the target nucleic acids and to prevent passing of the target nucleic acids.

The filter 104 is shaped to be a quadrangular pyramid having an open bottom surface and one open side surface. The open bottom surface of the pyramid is faced to the separation chamber 108. The open side surface of the pyramid is an upwardly opened horizontal surface. Accordingly, the target nucleic acids in a part of the filter 104 toward the separation chamber 108 can be sampled through the nozzle 110. As the target nucleic acids migrate toward the electrode 106 by the electrophoresis, the target nucleic acids are concentrated in a part of the filter 104 toward the electrode 106.

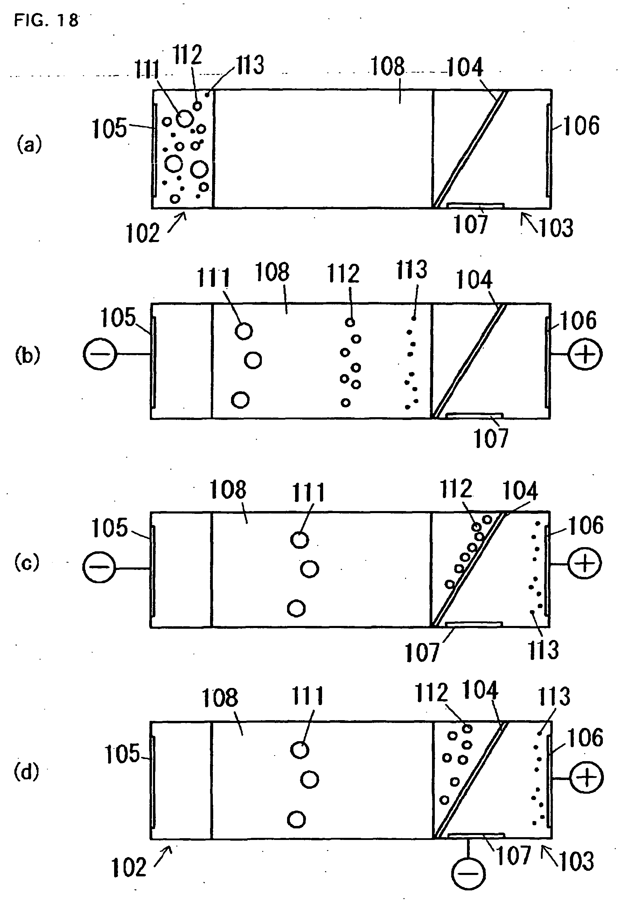

Next, explanation will be given of an embodiment of the present invention of concentration processes of the target nucleic acids by the concentration unit according to Fig. 18. Fig. 18 is a diagram of the concentration processes of the target nucleic acids. Firstly, Fig. 18 (a) shows the state that the sample is introduced into the injection chamber 102. For explaining plainly, herein, it is assumed that the sample contains target nucleic acids 112, nucleic acids 111 larger than the target nucleic acids 112 (bulkily or in molecular weight), and nucleic acids 113 smaller than the target nucleic acids 112.

Fig. 18 (a) shows the state that the large nucleic acids 111, the target nucleic acids 112 and the small nucleic acids 113 are mixed. When voltage is impressed on the electrodes 105 and 106, the large nucleic acids 111, the target nucleic acids 112 and the small nucleic acids 113 are introduced into the separation chamber 108, and migrate at different speeds in the separation chamber 108 as shown in Fig. 18 (b). With regard to the construction shown in Fig. 18, agarose gel is used as the separation medium filling the separation chamber 108, so as to make the small nucleic acids 113 precede the others.

The preceding small nucleic acids 113 reach the sampling chamber 103 through the separation chamber 108. Then, the small nucleic acids 113 pass through the filter 104 and migrate to the electrode 106 in the sampling chamber 103. Subsequently, the target nucleic acids 112 reach the sampling chamber 103 through the separation chamber 108. Then, as shown in Fig. 18 (c), the filter 104 prevents the target nucleic acids 112 from further migrating toward the electrode 106. Since the filter 104 is shaped in a pyramid whose open bottom thereof faces to the separation chamber 108 and whose one open side surface is directed upward, the target nucleic acids 112 are concentrated in the part of the filter 104 toward the electrode 106.

At this state, the voltage impression across the electrodes 105 and 106 is stopped and voltage is impressed across the electrodes 107 and 106 as shown in Fig. 18 (d). As a result, the electrode 106 still traps impurities, which are smaller than the target nucleic acids. When the voltage impression across the electrodes is stopped, the nucleic acids are liberated from the filter. In this way, during the liberation of the target nucleic acids 112 from the filter, and the small impurities and the small nucleic acids 113 are prevented from diffusing, thereby highly-accurately concentrating the target nucleic acids 112.

Then, the time for the target nucleic acids 112 to pass through the separation chamber 108 is calculated so as to determine the timing counted from the injection of the sample into the injection chamber 102 for shift of the voltage impression from that across the electrodes 105 and 106 to that across the electrodes 107 and 106, thereby automatically performing separation and concentration of the target nucleic acids 112.

Any gel for electrophoresis, such as agarose gel, can be used as the separation chamber 108. A separation medium used as a column filler can also be used. For example, a carrier for gel filtration, such as Sephadex (registered trademark) (Pharmacia), can be used. It also is considerable that gel for electrophoresis and a column filler are combined at the separation part 108 and adjusted so that the target nucleic acids flow out firstly.

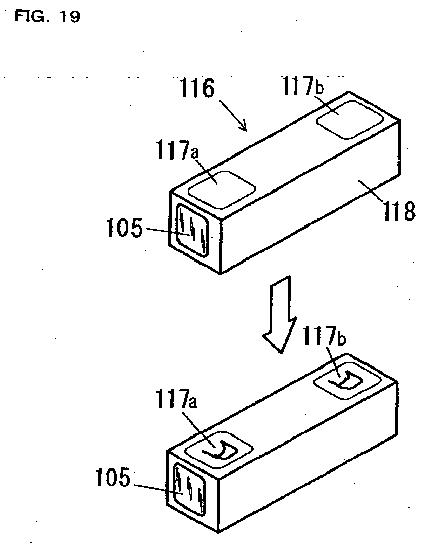

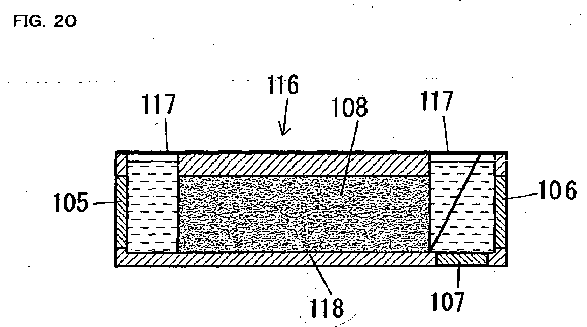

Next, explanation will be given of another construction of the concentration unit according to Figs. 19 and 20. Fig. 19 is a diagram of opening processes of a concentration unit, and Fig. 20 is a sectional side view of the concentration unit. The concentration unit is inserted into a test instrument so as to separate and concentrate the target nucleic acids.

Films 117a and 117b are stuck on an upper surface of the concentration unit 116, and the electrodes 105 and 106 are exposed on side surfaces of the concentration unit 116. The electrode 107 is exposed on a bottom surface thereof. The electrode 105 is connected to the injection chamber, and the electrodes 106 and 107 are connected to the sampling chamber. The concentration unit 116 holds the separation chamber 108 therein, and the injection chamber and the sampling chamber are filled with buffer for electrophoresis. The sampling chamber is separated by the filter.

In this state, the concentration unit 116 is sealed and voltage can be impressed thereon from the outside. Accordingly, the concentration unit can be handled easily. When a sample is injected into the concentration unit 116 so as to sample target nucleic acids, the films 117a and 117b are torn as shown in Fig. 19 and the sample is injected so as to sample the target nucleic acids. Since the films 117a and 117b are stuck on the upper surface of the concentration unit, any large shock is not given to the separation chamber 108 at the time of tearing the films 117, whereby the concentration and separation can be performed stably.

### Industrial Applicability

As the above, a method of the present invention simplifies operation for concentration and purification of a nucleic acid, and an apparatus for concentration and purification of a nucleic acid according to the present invention is simple in structure, thereby being applicable to an automatic test apparatus for concentrating and testing nucleic acids.

## Claims

1. A method for concentration and purification of a nucleic acid using gel electrophoresis, **characterized in that** an impurity (2) in a sample containing nucleic acids (111, 112, 113) is electrophoresed so as to contact the nucleic acid (111, 112, 113) with a separation medium (108) formed of material in which the nucleic acids (111, 112, 113) having different molecular weights migrate at different rates corresponding to the difference of molecular weights, and then a target nucleic acid (112) is recovered by filter (104), through which nucleic acids (113) tat are smaller than the target nucleic acid (112) can pass.

## Patentansprüche

1. Verfahren zum Anreichern und zur Aufreinigung einer Nukleinsäure mittels Gelelektrophorese,
**dadurch gekennzeichnet,**
**dass** eine Verunreinigung (2) in einer Probe, die Nukleinsäuren (111, 112, 113) enthält, so elektrophoresiert wird, dass die Nukleinsäure (111, 112, 113) mit einem Trennmedium (108) in Kontakt gebracht wird, das aus einem Material besteht, in dem die Nukleinsäuren (111, 112, 113), die verschiedene Molekulargewichte aufweisen, mit verschiedenen Raten entsprechend der Differenz der Molekulargewichte migrieren, und zudem eine Zielnukleinsäure (112) mit Hilfe eines Filters (104) gewonnen wird, durch den Nukleinsäuren (113), die kleiner als die Zielnukleinsäure (112) sind, passieren können.

## Revendications

1. Procédé pour la concentration et la purification d'un acide nucléique en utilisant une électrophorèse en gel,
**caractérisé en**
**ce qu'**une impureté (2) dans un échantillon contenant des acides nucléiques (111, 112, 113) subit une électrophorèse de manière à venir mettre en contact l'acide nucléique (111, 112, 113) avec un milieu de séparation (108) formé de matière dans laquelle les acides nucléiques (111, 112, 113) ayant différents poids moléculaires migrent à différentes vitesses correspondant à la différence de poids moléculaires, puis un acide nucléique cible (112) est récupéré par un filtre (104) à travers lequel les acides nucléiques (113) qui sont plus petits que l'acide nucléique cible (112) peuvent passer.
